(19) **Europäisches Patentamt European Patent Office Office européen des brevets**

(11) **EP 2 276 796 B1**

(12) **EUROPEAN PATENT SPECIFICATION**

(45) Date of publication and mention of the grant of the patent:
**05.12.2012 Bulletin 2012/49**

(51) Int Cl.:
*C08J 3/03* (2006.01)　　*A61K 8/892* (2006.01)
*A61K 8/06* (2006.01)　　*A61K 8/896* (2006.01)
*A61Q 5/12* (2006.01)　　*A61Q 19/00* (2006.01)
*C08J 3/05* (2006.01)

(21) Application number: **09733429.6**

(22) Date of filing: **13.04.2009**

(86) International application number:
**PCT/US2009/040359**

(87) International publication number:
**WO 2009/129179 (22.10.2009 Gazette 2009/43)**

(54) **EMULSIONS OF BORON CROSSLINKED ORGANOPOLYSILOXANES AND THEIR USE IN PERSONAL CARE COMPOSITIONS**

EMULSIONEN VON BORVERNETZTEN ORGANOPOLYSILOXANEN UND IHRE VERWENDUNG IN KÖRPERPFLEGEZUSAMMENSETZUNGEN

ÉMULSIONS D'ORGANOPOLYSILOXANES RÉTICULÉS PAR DU BORE ET LEUR UTILISATION DANS DES COMPOSITIONS DE SOINS PERSONNELLES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **22.12.2008 US 139708 P**
**14.04.2008 US 44594 P**

(43) Date of publication of application:
**26.01.2011 Bulletin 2011/04**

(73) Proprietor: **Dow Corning Corporation Midland, Michigan 48686-0994 (US)**

(72) Inventors:
• **CAUVIN, Séverine**
**7000 Mons (BE)**
• **GORDON, Glenn**
**Midland**
**MI 48642 (US)**
• **JOHNSON, Bethany**
**Midland**
**MI 48642 (US)**
• **LILES, Donald**
**Midland**
**MI 48642 (US)**

(74) Representative: **Gillard, Richard Edward et al Elkington and Fife LLP Thavies Inn House 3-4 Holborn Circus London EC1N 2HA (GB)**

(56) References cited:
**EP-A- 0 446 157　　US-A- 4 863 985**

EP 2 276 796 B1

**Description**

<u>Cross Reference to Related Applications</u>

This application claims the benefit of U.S. Provisional Patent Application No. 61/044594 filed April 14, 2008 and U.S. Provisional Patent Application 61/139708 filed December 22, 2008.

<u>Technical Field</u>

This disclosure relates to emulsion compositions of boron crosslinked silicones. The emulsions provide coatings of high viscosity silicones which are useful in various personal care products.

<u>Background</u>

There have been numerous attempts to provide emulsions of high molecular weight silicones as an alternative to handling such materials in bulk. Generally, there are two types of processes to prepare emulsion of high molecular weight silicones, either via emulsion polymerization of siloxane monomers or mechanical emulsification of pre-formed high molecular weight silicones. Very often, there is a need to incorporate other components in a silicone composition to affect the properties of the silicone. For example, other additive components such as fillers, catalysts, or crosslinkers may be of interest in a silicone composition to alter subsequent physical properties of the silicone. However, incorporation of many of these components in a silicone emulsion composition may be problematic depending on the selection of the additive component and the emulsion process (that is mechanical vs emulsion polymerization process). For example, if the desired silicone additive component interacts with water during the emulsification process it may be difficult or impossible to incorporate its addition into a silicone emulsion.

There are abundant examples in the silicone emulsion art incorporating a variety of crosslinking components with organopolysiloxanes to provide high molecular weight or elastomeric silicone compositions. However, there are few examples for the addition of boron compounds as crosslinking additives in a silicone emulsion.

Many boron crosslinked organopolysiloxanes, or borosiloxanes, are of particular interest because of their inherent dilatant properties. For example, dilatant boron crosslinked silicones are used in active protection systems (APS) wherein a fabric is coated with a boron crosslinked silicone. However, such processes require the boron crosslinked silicone be first dispersed or dissolved in a solvent. Water based emulsions of boron crosslinked silicones would be easier to use and environmentally more desirable in such processes. However, there are few examples of emulsions of boron crosslinked silicones or borosiloxanes. Presumably, the interaction of a boron crosslinker with water has limited this crosslinking chemistry in a water based composition. Thus, a need exists to provide emulsions of boron crosslinked silicones to avoid undesirable solvent based processes.

U.S. 4,863,985 teaches aqueous silicone emulsions cross-linkable into an elastomeric state. In particular, U.S. 4,863,985 discloses thixotropic silicone emulsions cross-linkable into elastomeric state upon removal of water therefrom, e.g., to fabricate elastomer seals for the construction industry, have a pH of from 4 to 8 and a solids content of at least 50%, and contain: (A) 100 parts of an oil-in-water emulsion of an $\alpha,\omega$-(dihydroxy) polydiorganosiloxane, and a stabilizing amount of at least one anionic or nonionic surface-active agent, or mixture thereof; (B) 1 to 15 parts of a siliceous reinforcing filler in powder form; (C) 0 to 250 parts of an inorganic filler other than the siliceous filler (B); (D) 0.01 to 2 parts of a catalytic tin compound; and (E) 0.1 to 5 parts of boric oxide, a boric acid or a borate. While U.S. 4,863,985 teaches emulsions of silicones that provide elastomeric silicones, the addition of tin and fillers are necessary in these compositions. Furthermore, U.S. 4,863,985 teaches the addition of a boron compound to a pre-formed oil-in-water emulsion of $\alpha,\omega$-(dihydroxy) polydiorganosiloxane. EP 0 446 157 discloses aqueous dispersions based on silicone able to crosslink to form a flame-resistant elastomer, by elimination of water. The aqueous dispersions comprise an emulsion (A), of the oil-in-water type, of an $\alpha,\omega$-(dihydroxy)polydiorganosiloxane, stabilised by at least one surfactant, at least one crosslinking agent, (B), a non-siliceous inorganic filler, $(C_1)$, borax hydrate powder, $(C_2)$, and optionally, a catalystic hardening metal compound.

The present inventors have discovered emulsions of boron crosslinked organopolysiloxanes. In contrast to prior art teachings, the present inventors have unexpectedly discovered that boron compounds may be incorporated with an organopolysiloxane prior to forming an emulsion. The process provides emulsions of boron crosslinked organopolysiloxanes that yield subsequent silicone polymers of varying physical properties. In particular, the present process provides emulsions of boron crosslinked organopolysiloxanes that yield coatings of a highly viscous silicone which may be useful in various personal care products, and in particular for hair care applications to provide conditioning benefits, which include ease of detangling, combing, pliability, smoothness and slipperiness.

## Summary

This disclosure provides emulsions of boron crosslinked organopolysiloxanes having a viscosity of at least 1000 mPa·s at 24°C wherein the emulsion is obtainable by:

I) forming a mixture of;

A) a silanol functional organopolysiloxane,
B) a boron compound,
C) an emulsifier,

II) admixing water to the mixture from step I) to form an emulsion,
III) optionally, further shear mixing the emulsion.

The present emulsions are useful in a variety of applications. For example, the present process provides emulsions of high viscosity silicones that are useful in personal care products, and in particular they provide conditioning benefits when formulated in hair care products.

## Detailed Description

The first step of the process to prepare the present emulsions involves forming a mixture of;

A) a silanol functional organopolysiloxane,
B) a boron compound, and
C) an emulsifier,

each of which are described in more detail as follows.

### A) The Silanol Functional Organopolysiloxane

Organopolysiloxanes are polymers containing siloxane units independently selected from $(R_3SiO_{0.5})$, $(R_2SiO)$, $(RSiO_{1.5})$, or $(SiO_2)$ siloxy units, where R may independently be an organic group, OH (silanol), or H (SiH functional). These siloxy units are commonly referred to as M, D, T, and Q units respectively. These siloxy units can be combined in various manners to form cyclic, linear, or branched structures. The chemical and physical properties of the resulting polymeric structures will vary depending on the type and number of each siloxy units present in the organopolysiloxane. For example organopolysiloxanes can be volatile or low viscosity fluids, high viscosity fluids/gums, elastomers or rubbers, and resins.

The organopolysiloxane useful as component A) in the present invention may have any combination of $(R_3SiO_{0.5})$, $(R_2SiO)$, $(RSiO_{1.5})$, or $(SiO_2)$ siloxy units, providing the organopolysiloxane contains at least one silanol group (SiOH). Thus, the organopolysiloxane may have varying molecular weights and be a liquid, a gum, an elastomer, a resin, or any combination thereof. For example, the organopolysiloxane may be a mixture of a higher molecular weight organopolysiloxane (such as an elastomer or resin) in a lower molecular weight liquid organopolysiloxane, providing there is at least one silanol group in the organopolysiloxane composition of component A.

The amount of silanol groups present in the organopolysiloxane may vary. The amount of silanol groups in the organopolysiloxane may be designated as weight percent of SiOH. The weight percent of silanol groups that are typical in the organopolysiloxanes useful as component A) vary from 0.01 to 20 weight percent, alternatively from 0.05 to 10 weight percent, alternatively from 0.05 to 4 weight percent.

In one embodiment, the organopolysiloxane is a predominately linear polydimethylsiloxane having terminal silanol groups. The predominately linear polydimethylsiloxane having terminal silanol groups may have the formula;

$$HO(Me)_2SiO[(Me)_2SiO]_x(Me)_2SiOH$$

where x is > 0, alternatively, x is 1 - 4000, alternatively 10 - 1000.

In yet another embodiment, the silanol functional organopolysiloxane may be mixed with other silane or polysiloxane components before or during mixing with components B) and C). The other silane or siloxane components include organofunctional silanes or organofunctional polysiloxanes that can react with the silanol functional organopolysiloxane. Suitable organofunctional silanes include amino functional silanes such as;
aminopropyl trimethoxysilane,

ethylenediaminepropyl trimethoxysilane, or
ethylenediamineisobutyl trimethoxysilane.

Suitable organofunctional polysiloxanes include amino functional organopolysiloxanes such as those having a formula

$$R^2 R_2SiO(R_2SiO)_a (R^1 RSiO)_b SiR_2 R^2$$

or

$$R^2 R_2 SiO(R_2 SiO)_a (R^1 SiO_{3/2})_b SiR_2 R^2$$

wherein R is a monovalent organic group, $R^1$ is an aminoalkyl group having its formula selected from the group consisting of $-R^3 NH_2$ and $-R^3 NHR^4 NH_2$ wherein $R^3$ is a divalent hydrocarbon group having at least 3 carbon atoms and $R^4$ is a divalent hydrocarbon group having at least 2 carbon atoms, $R^2$ is R, $R^1$, or OH, a has a value of 0 to 2000, and b has a value of from greater than zero to 200. The monovalent R groups are exemplified by alkyl groups such as the methyl, ethyl, propyl, butyl, amyl, and hexyl; alkenyl groups such as the vinyl, allyl, and hexenyl; cycloalkyl groups such as the cyclobutyl and cyclohexyl; aryl groups such as the phenyl and naphthyl; aralkyl groups such as the benzyl and 2-phenylethyl; alkaryl groups such as the tolyl, and xylyl; halohydrocarbon groups such as 3-chloropropyl, 4-bromobutyl, 3,3,3-trifluoropropyl, chlorocyclohexyl, bromophenyl, and chlorophenyl. Typically R is a monovalent hydrocarbon group having from 1 to 6 carbon atoms. Especially preferred R groups are methyl, phenyl, and vinyl. The group $R^3$ is typically an alkylene group having from 3 to 20 carbon atoms. Typically $R^3$ is selected from propylene, $-CH_2 CHCH_3 -$, butylene, $-CH_2 CH(CH_3)CH_2 -$, pentamethylene, hexamethylene, 3-ethyl-hexamethylene, octamethylene, and decamethylene. The group $R^4$ is typically an alkylene group having from 2 to 20 carbon atoms. Typically $R^4$ is selected from ethylene, propylene, $-CH_2 CHCH_3 -$, butylene, $-CH_2 CH(CH_3)CH_2 -$, pentamethylene, hexamethylene, 3-ethyl-hexamethylene, octamethylene, and decamethylene. $R^1$ typically is $-CH_2 CH_2 CH_2 NHCH_2 CH_2 NH_2$ or $--CH_2 CH(CH_3)CH_2 NHCH_2 CH_2 NH_2$. Salts of these same aminofunctional groups may also be used. Examples of such salts include alkyl carboxylate salts, aryl carboxylate salts, halide salts such as chlorides and bromides, and other neutralization products of the amines with organic acids. Although the group $R^2$ may be R, $R^1$, or -OH, typically $R^2$ is methyl or -OH. The polyorganosiloxanes may have from 0.1 to 15 molar percent of the above described amino groups and most typically from 0.2 to 10 molar percent of the above described amino groups. In the above formulas, typically a has a value of from 50 to 2000, and b has a value of 1 to 100. The aminofunctional polyorganosiloxanes useful in this invention can be prepared by procedures well known in the art. Many of these polyorganosiloxanes are available commercially.

The amount of the silanol functional organopolysiloxane added in step I may vary. The amount used will depend on the type and amount of boron compound used in step I) and the extent of crosslinking desired. Typically, the amount of the silanol functional organopolysiloxane ranges from 50 to 99, alternatively from 75 to 95, alternatively from 85 to 90 weight, percent based on the total weight of the mixture in step I.

### B) The Boron Compound

Component B) is a boron compound. As used herein, a "boron compound" means any compound containing boron. Any boron compound known to react with organopolysiloxanes may be selected as component B). Alternatively, the boron compound may be selected from those known to react with silanol functional groups on organopolysiloxanes. Such boron compounds include; boron or boric oxide, boric acid, borates, boric anhydride. Boric acid may be orthoboric acid, metaboric acid, or tetraboric acid. Borates include alkyl and allyl boric acid esters / triorganoborates that hydrolyse to boric acid in the presence of water, such as triethylborate, triphenylborate, tribenzyl borate, tricyclohexyl borate, tri (methylsilyl) borate, tri-t-butyl borate, trialkoxyboroxines such as trimethoxyboroxine and triisopropoxyboroxine, triethanolamineborate, and derivatives such as 2,2'-oxybis[4,4,6-trimethyl-1,3,2-dioxaboranine. Borates also encompass inorganic borates such as diammonium pentaborate, sodium tetraborate decahydrate (borax), potassium pentaborate, magnesium diborate, calcium monoborate, barium triborate, and zinc metaborate. Suitable boron compounds further includes the partial hydrolysis products of the aforementioned borates. Typically, the boron compound is boron oxide having the formula $B_2O_3$ [CAS registry #1303-86-2], which is also known as boron (III) oxide, boron trioxide, or diboron trioxide.

The boron compound may be added either alone or in combination with other ingredients in step I.

In one embodiment, the boron compound is boron oxide, and is dispersed in a trimethylsiloxy terminated polydimethylsiloxane, such as Dow Corning ® 200 fluid having a viscosity ranging from 0.65 cS (mm²/s) at 25°C to 100,000 cS (mm²/s) at 25 °C, alternatively from 100 to 10,000 cS (mm²/s) at 25 °C, or alternatively from 100 to 1,000 cS (mm²/s) at 25 °C.

The amount of boron oxide dispersed in the trimethylsiloxy terminated polydimethylsiloxane may vary, but typically ranges from 0.5 to 70, alternatively from 10 to 50 weight percent alternatively from 40 to 50 weight percent.

The amount of boron compound added in step I may vary. The amount used will depend on the type and amount of organopolysiloxane used in step I) and the extent of crosslinking desired. Typically, the amount of boron compound ranges from 0.05 to 30, alternatively from 0.1 to 10 alternatively from 0.1 to 6 weight percent based on the total weight of the mixture in step I.

C) *The Emulsifier*

Component C in the process of the present disclosure is an emulsifier. As used herein, "emulsifier" refers to any compound or substance that enables the formation of an emulsion. The emulsion may be an oil/water emulsion, a water/oil emulsion, a multiple phase or triple emulsion. The emulsifier may be selected from any surface active compound or polymer capable of stabilizing emulsions. Typically, such surface active compounds or polymers stabilize emulsions by preventing coalescence of the dispersed particles. The surface active compounds useful as emulsifiers in the present process may be a surfactant or combination of surfactants. The surfactant may be an anionic surfactant, cationic surfactant, nonionic surfactant, amphoteric surfactant, or a mixture of any of these surfactants.

Representative examples of suitable anionic surfactants include alkali metal soaps of higher fatty acids, alkylaryl sulphonates such as sodium dodecyl benzene sulphonate, long chain fatty alcohol sulphates, olefin sulphates and olefin sulphonates, sulphated monoglycerides, sulphated esters, sulphonated ethoxylated alcohols, sulphosuccinates, alkane sulphonates, phosphate esters, alkyl isethionates, alkyl taurates, and alkyl sarcosinates.

Representative examples of suitable cationic surfactants include alkylamine salts, quaternary ammonium salts, sulphonium salts, and phosphonium salts. Representative examples of suitable nonionic surfactants include condensates of ethylene oxide with long chain fatty alcohols or fatty acids such as a $C_{12-16}$ alcohol, condensates of ethylene oxide with an amine or an amide, condensation products of ethylene and propylene oxide, esters of glycerol, sucrose, sorbitol, fatty acid alkylol amides, sucrose esters, fluoro-surfactants, and fatty amine oxides. Representative examples of suitable amphoteric surfactants include imidazoline compounds, alkylaminoacid salts, and betaines.

Representative examples of suitable commercially available nonionic surfactants include polyoxyethylene fatty alcohols sold under the tradename BRIJ® by Uniqema (ICI Surfactants), Wilmington, Delaware. Some examples are BRIJ® 35 Liquid, an ethoxylated alcohol known as polyoxyethylene (23) lauryl ether, and BRIJ® 30, another ethoxylated alcohol known as polyoxyethylene (4) lauryl ether. Some additional nonionic surfactants include ethoxylated alcohols sold under the trademark TERGITOL® by The Dow Chemical Company, Midland, Michigan. Some example are TERGITOL® TMN-6, an ethoxylated alcohol known as ethoxylated trimethylnonanol; and various of the ethoxylated alcohols, i.e., $C_{12}$-$C_{14}$ secondary alcohol ethoxylates, sold under the trademarks TERGITOL® 15-S-5, TERGITOL® 15-S-12, TERGITOL® 15-S-15, and TERGITOL® 15-S-40. Lutensol ® supplied by BASF in the series of Lutensol XP known as ethoxylated, C10-Guerbet alcohol and Lutensol TO known as ethoxylated, iso-C13 alcohol may also be used.

When mixtures containing nonionic surfactants are used, one nonionic surfactant may have a low Hydrophile-Lipophile Balance (HLB) and the other nonionic surfactant may have a high HLB, such that the two nonionic surfactants have a combined HLB of 11 -15, alternatively a combined HLB of 12.5-14.5.

Alternatively, the emulsifier may be a polymer or those materials consider as "thickeners" or "thickening agents". Such polymeric emulsifiers include polyvinyl alcohol, cellulosic polymers or xanthan gums. The polyvinyl alcohol includes hydrolyzed polyvinyl alcohols, such as 80 - 95 % hydrolyzed polyvinyl alcohol. Suitable thickening agents are exemplified by sodium alginate, gum arabic, polyoxyethylene, guar gum, hydroxypropyl guar gum, ethoxylated alcohols, such as laureth-4 or polyethylene glycol 400, cellulose derivatives exemplified by carboxy methylcellulose, methylcellulose, methylhydroxypropylcellulose, hydroxypropylcellulose, polypropylhydroxyethylcellulose, starch, and starch derivatives exemplified by hydroxyethylamylose and starch amylose, locust bean gum, electrolytes exemplified by sodium chloride and ammonium chloride, and saccharides such as fructose and glucose, and derivatives of saccharides such as PEG-120 methyl glucose diolate or mixtures of 2 or more of these. Typically the thickening agent is selected from the group consisting of cellulose derivatives, saccharide derivatives, and electrolytes, or from a combination of two or more of the above thickening agents exemplified by a combination of a cellulose derivative and any electrolyte, and a starch derivative and any electrolyte.

The emulsifier may be added either alone or in combination with varying amounts of water in step I. Typically, when a surfactant or surfactant combination is selected as the emulsifier, the surfactant is added in step I as a concentrated aqueous dispersion, or alternatively as an aqueous solution.

In one embodiment, the emulsifier is an aqueous solution containing at least 70 weight percent of two nonionic surfactants having a combined HLB of 8 -15.

Alternatively the emulsifier is an aqueous solution of 5 to 30 weight percent of nonionic surfactant having alone a HLB of 8 to 15, or the emulsifier is an aqueous solution containing at least 20 weight percent of one nonionic surfactant and a cationic surfactant, or the emulsifier is an aqueous surfactant containing 30 to 100 weight percent of a anionic surfactant.

The amount of emulsifier added in step I may vary. The amount used will depend on the type of emulsion and the particle size desired. Typically, the amount of emulsifier added in Step I is 0.1 to 40, alternatively the amount is 0.5 to 30 weight percent of the mixture in Step I.

Mixing in step (I) can be accomplished by any method known in the art to affect mixing of high viscosity materials. The mixing may occur either as a batch, semi-continuous, or continuous process. Mixing may occur, for example using, batch mixing equipments with medium / low shear include change-can mixers, double-planetary mixers, conical-screw mixers, ribbon blenders, double-arm or sigma-blade mixers; batch equipments with high-shear and high-speed dispersers include those made by Charles Ross & Sons (NY), Hockmeyer Equipment Corp. (NJ); batch equipments with high shear actions include Banbury-type (CW Brabender Instruments Inc., NJ) and Henschel type (Henschel mixers America, TX). Illustrative examples of continuous mixers / compounders include extruders single-screw, twin-screw, and multi-screw extruders, co-rotating extruders, such as those manufactured by Krupp Werner & Pfleiderer Corp (Ramsey, NJ), and Leistritz (NJ); twin-screw counter-rotating extruders, two-stage extruders, twin-rotor continuous mixers, dynamic or static mixers or combinations of these equipments.

The temperature and pressure at which the mixing of step I occurs is not critical, but generally is conducted at ambient temperature and pressures. Typically, the temperature of the mixture will increase during the mixing process due to the mechanical energy associated with shearing such high viscosity materials.

Although not wishing to be bound by any theory, the present inventors believe that as a result of mixing components A), B), and C) the boron compound reacts with the silanol functional organopolysiloxane to form various crosslinks. However, the inclusion of an emulsifier in the step I) mixture enhances subsequent emulsion formation in step II).

Step II of the process involves admixing water to the mixture of step I to form an emulsion. Typically 5 to 700 parts water are mixed for every 100 parts of the step I mixture to form an emulsion. In one embodiment the emulsion formed is a water continuous emulsion. Typically, the water continuous emulsion has dispersed particles of the boron crosslinked organopolysiloxane from step I, and having an average particle size less than 150 $\mu$m.

The amount of water added can vary from 5 to 700 parts per 100 parts by weight of the mixture from step I. The water is added to the mixture from step I at such a rate so as to form an emulsion of the mixture of step I. While this amount of water can vary depending on the selection of the boron oxide crosslinked organopolysiloxane and emulsifier, generally the amount of water is from 5 to 700 parts per 100 parts by weight of the step I mixture, alternatively from 5 to 100 parts per 100 parts by weight of the step I mixture, or alternatively from 5 to 70 parts per 100 parts by weight of the step I mixture.

Typically the water is added to the mixture from step I in incremental portions, whereby each incremental portion comprises less than 30 weight % of the mixture from step I and each incremental portion of water is added successively to the previous after the dispersion of the previous incremental portion of water, wherein sufficient incremental portions of water are added to form an emulsion of the boron oxide crsslinked organopolysiloxane.

Mixing in step (II) can be accomplished by any method known in the art to affect mixing of high viscosity materials. The mixing may occur either as a batch, semi-continuous, or continuous process. Any of the mixing methods as described for step (I), may be used to affect mixing in step (II).

Optionally, the water continuous emulsion formed in step (II) may be further sheared according to step (III) to reduce particle size and/or improve long term storage stability. The shearing may occur by any of the mixing techniques discussed above.

The emulsion products resulting from the present process may be an oil/water emulsion, a water/oil emulsion, a multiple phase or triple emulsion.

In one embodiment, the emulsion products produced by the present process are oil/water emulsions. The oil/ water emulsion may be characterized by average volume particle of the dispersed boron crosslinked organopolysiloxane phase in a continuous aqueous phase. The particle size may be determined by laser diffraction of the emulsion. Suitable laser diffraction techniques are well known in the art. The particle size is obtained from a particle size distribution (PSD). The PSD can be determined on a volume, surface, length basis. The volume particle size is equal to the diameter of the sphere that has the same volume as a given particle. The term Dv represents the average volume particle size of the dispersed particles. Dv 0.5 is the particle size measured in volume corresponding to 50% of the cumulative particle population. In other words if Dv 0.5 = 10 $\mu$m, 50% of the particle have an average volume particle size below 10 $\mu$m and 50% of the particle have a volume average particle size above 10 $\mu$m. Unless indicated otherwise all average volume particle sizes are calculated using Dv 0.5.

The average volume particle size of the dispersed siloxane particles in the oil/water emulsions is between 0.1 $\mu$m and 150 $\mu$m;

or between 0.1 $\mu$m and 30 $\mu$m;

or between 0.3 $\mu$m and 5.0 $\mu$m.

The emulsions of the present disclosure may be further characterized by the properties of the resulting films or coatings produced after allowing a film of the emulsion to dry. Typically, such coatings are obtained by forming a film of the emulsion, and allowing the film to stand for a sufficient period of time to evaporate the water present in the emulsion. This process may be accelerated by increasing the ambient temperature of the film or coating.

The films or coatings resulting from the present emulsions may by characterized by their rheological properties, such as with a Carri-Med rheometer to determine in the LVR at 0.2 Hz both the dynamic shear storage (G') and loss module (G").

In one embodiment, the resulting films may be considered as high viscosity fluids as characterized by dynamic viscosity. The dynamic viscosity of the resulting films are at least 1000 mPa·s, alternatively from 10,000 mPa·s to 100,000,000 mPa·s, or alternatively from 10,000 mPa·s to 10,000,000 mPa·s, as measured at 24 °C.

Other additives can also be incorporated in the emulsions of the present disclosure, such as fillers, foam control agents; anti-freeze agents and biocides.

Compositions comprising the silicone emulsions may be formulated into personal care products. The personal care compositions of this invention may be in the form of a cream, a gel, a powder, a paste, or a freely pourable liquid. Generally, such compositions can generally be prepared at room temperature if no solid materials at room temperature are presents in the compositions, using simple propeller mixers, Brookfield counter-rotating mixers, or homogenizing mixers. No special equipment or processing conditions are typically required. Depending on the type of form made, the method of preparation will be different, but such methods are well known in the art.

The personal care products may be functional with respect to the portion of the body to which they are applied, cosmetic, therapeutic, or some combination thereof. Conventional examples of such products include, but are not limited to: antiperspirants and deodorants, skin care creams, skin care lotions, moisturizers, facial treatments such as acne or wrinkle removers, personal and facial cleansers, shower gels, bath oils, perfumes, colognes, sachets, sunscreens, pre-shave and after-shave lotions, shaving soaps, and shaving lathers, hair shampoos, hair conditioners, hair colorants, hair relaxants, hair sprays, mousses, gels, permanents, depilatories, and cuticle coats, make-ups, color cosmetics, foundations, concealers, blushes, lipsticks, eyeliners, mascara, oil removers, color cosmetic removers, and powders, medicament creams, pastes or sprays including anliacne, dental hygienic, antibiotic, healing promotive, nutritive and the like, which may be preventative and/or therapeutic. In general the personal care products may be formulated with a carrier that permits application in any conventional form, including but not limited to liquids, rinses, lotions, creams, pastes, gels, foams, mousses, ointments, sprays, aerosols, soaps, sticks, soft solids, solid gels, and gels. What constitutes a suitable carrier is readily apparent to one of ordinary skill in the art.

The present compositions can be used in a variety of personal, household, and healthcare applications. In particular, the compositions of the present invention may be used in the personal care products as taught in US Patent Nos. 6,051,216, 5,919,441, 5,981,680; as disclosed in WO 2004/060271 and WO 2004/060101; in sunscreen compositions as taught in WO 2004/060276; in cosmetic compositions also containing film-forming resins, as disclosed in WO 03/105801; in the cosmetic compositions as taught in US Patent Application Publications 2003/0235553, 2003/0072730, 2003/0170188, EP 1,266,647, EP 1,266,648, EP1,266,653, WO 03/105789, WO 2004/000247 and WO 03/106614; as additional agents to those taught in WO 2004/054523; in long wearing cosmetic compositions as taught in US Patent Application Publication 2004/0180032; in transparent or translucent care and/or make up compositions as discussed in WO 2004/054524.

The compositions according to this invention can be used by the standard methods, such as applying them to the human body, e.g. skin or hair, using applicators, brushes, applying by hand, pouring them and/or possibly rubbing or massaging the composition onto or into the body. Removal methods, for example for color cosmetics are also well known standard methods, including washing, wiping, peeling and the like. For use on the skin, the compositions according to the present invention may be used in a conventional manner for example for conditioning the skin. An effective amount of the composition for the purpose is applied to the skin. Such effective amounts generally range from about 1 mg/cm$^2$ to about 3 mg/cm$^2$. Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The use of the compositions according to the invention on hair may use a conventional manner for conditioning hair. An effective amount of the composition for conditioning hair is applied to the hair. Such effective amounts generally range from about 0.5 g to about 50 g, preferably from about 1 g to about 20 g. Application to the hair typically includes working the composition through the hair such that most or all of the hair is contacted with the composition. This method for conditioning the hair comprises the steps of applying an effective amount of the hair care composition to the hair, and then working the composition through the hair. These steps can be repeated as many times as desired to achieve the desired conditioning benefit.

Non-limiting examples of additives which may be formulated into the personal care compositions in addition to the boron crosslinked organopolysiloxane emulsions include: additional silicones, anti-oxidants, cleansing agents, colorants, additional conditioning agents, deposition agents, electrolytes, emollients and oils, exfoliating agents, foam boosters, fragrances, humectants, occlusive agents, pediculicides, pH control agents, pigments, preservatives, biocides, other solvents, stabilizers, sun-screening agents, suspending agents, tanning agents, other surfactants, thickeners, vitamins, botanicals, fragrances, waxes, rheology-modifying agents, anti-dandruff, anti-acne, anti-carie and wound healing-promotion agents.

The personal care composition, such as a shampoo or cleanser may contain at least one anionic detersive surfactant. This can be any of the well-known anionic detersive surfactants typically used in shampoo formulations. These anionic detersive surfactants function as cleansing agents and foaming agents in the shampoo compositions of this invention. The anionic detersive surfactants are exemplified by alkali metal sulfonates, sulfonated glyceryl esters of fatty acids such as sulfonated monoglycerides of coconut oil acids, salts of sulfonated monovalent alcohol esters such as sodium oleylisethianate, amides of amino sulfonic acids such as the sodium salt of oleyl methyl tauride, sulfonated products of fatty acids nitriles such as palmitonitrile sulfonate, sulfonated aromatic hydrocarbons such as sodium alpha-naphthalene monosulfonate, condensation products of naphthalene sulfonic acids with formaldehyde, sodium octahydroanthracene sulfonate, alkali metal alkyl sulfates such as sodium lauryl sulfate, ammonium lauryl sulfate or triethanol amine lauryl sulfate, ether sulfates having alkyl groups of 8 or more carbon atoms such as sodium lauryl ether sulfate, ammonium lauryl ether sulfate, sodium alkyl aryl ether sulfates, and ammonium alkyl aryl ether sulfates, alkylarylsulfonates having 1 or more alkyl groups of 8 or more carbon atoms, alkylbenzenesulfonic acid alkali metal salts exemplified by hexylbenzenesulfonic acid sodium salt, octylbenzenesulfonic acid sodium salt, decylbenzenesulfonic acid sodium salt, dodecylbenzenesulfonic acid sodium salt, cetylbenzenesulfonic acid sodium salt, and myristylbenzenesulfonic acid sodium salt, sulfuric esters of polyoxyethylene alkyl ether including $CH_3(CH_2)_6CH_2O(C_2H_4O)_2SO_3H$, $CH_3(CH_2)_7CH_2O(C_2H_4O)_{3.5}SO_3H$, $CH_3(CH_2)_8CH_2O(C_2H_4O)_8SO_3H$, $CH_3(CH_2)_{19}CH_2O(C_2H_4O)_4SO_3H$, and $CH_3(CH_2)_{10}CH_2O(C_2H_4O)_6SO_3H$, sodium salts, potassium salts, and amine salts of alkylnaphthylsulfonic acid. Preferably the detersive surfactant is selected from the group consisting of sodium lauryl sulfate, ammonium lauryl sulfate, triethanolamine lauryl sulfate, sodium lauryl ether sulfate, and ammonium lauryl ether sulfate. The anionic detersive surfactant is present in the shampoo compositions of this invention in an amount from about 5 to 50 wt% and preferably about 5 to 25 wt% based on the total weight of the composition.

The personal care composition may contain at least one cationic deposition aid, preferably a cationic deposition polymer. The cationic deposition aid will generally be present at levels of from 0.001 to 5%, preferably from about 0.01 to 1 %, more preferably from about 0.02% to about 0.5% by weight. The polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof. The cationic charge density has been found to need to be at least 0.1 meq/g, preferably above 0.8 or higher. The cationic charge density should not exceed 4 meq/g, it is preferably less than 3 and more preferably less than 2 meq/g. The charge density can be measured using the Kjeldahl method and should be within the above limits at the desired pH of use, which will in general be from about 3 to 9 and preferably between 4 and 8. The cationic nitrogen-containing group will generally be present as a substituent on a fraction of the total monomer units of the cationic deposition polymer. Thus when the polymer is not a homopolymer it can contain spacer noncationic monomer units. Such polymers are described in the CTFA Cosmetic Ingredient Directory, 3rd edition. Suitable cationic deposition aids include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyil substituted monomers preferably have CI-C7 alkyl groups, more preferably C1-3 alkyl groups. Other suitable spacers include vinyl esters, vinyl alcohol, maleic anhydride, propylene glycol and ethylene glycol. The cationic amines can be primary, secondary or tertiary amines, depending upon the particular species and the pH of the composition. In general secondary and tertiary amines, especially tertiary, a-re preferred. Amine substituted vinyl monomers and amines can be polymerized in the amine form and then converted to ammonium by quaternization. Suitable cationic amino and quaternary ammonium monomers include, for example, vinyl compounds substituted with dialkyl aminoalkyl acrylate, dialkylamino alkylmethacrylate, monoalkylaminoalkyl acrylate, monoalkylaminoalkyl methacrylate, trialkyl methacryloxyalkyl ammonium salt, triaikyi acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidine, e.g., alkyl vinyl imidazolium, and quaternized pyrrolidine, e.g., alkyl vinyl imidazolium, alkyl vinyl pyridinium, alkyl vinyl pyrrolidine salts. The alkyl portions of these monomers are preferably lower alkyls such as the C,-C., alkyls, more preferably C, and C2 alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C,-C,hydrocarbyls, more preferably C,-C,, alkyls. The cationic deposition aids can comprise mixtures of monomer units derived from amine- and/or quaternary ammonium-substituted monomer and/or compatible spacer monomers. Suitable cationic deposition aids include, for example: copolymers of 1-vinyl-2-pyrrolidine and 1-vinyl-3-methylimidazolium salt (e.g., Chloride salt) (referred to in the industry by the Cosmetic, Toiletry, and Fragrance Association, "CTFA". as Polyquaternium-16) such as those commercially available from BASF Wyandotte Corp. (Parsippany, NJ, USA) under the LUVIQUAT tradename (e.g., LUVIQUAT FC 370); copolymers of 1-vinyl-2-pyrrolidine and dimethylaminoethyl methacrylate (referred to in the industry by CTFA as Polyquaternium-11) such as those commercially from Gar Corporation (Wayne, NJ, USA) under the GAFQUAT tradename (e.g., GAFQUAT 755N); cationic diallyl quaternary ammonium-containing polymer including, for example, dimethyldiallyammonium chloride homopolymer and copolymers of acrylamide and dimethyl diallyammonium chloride, referred to in the industry (CTFA) as Polyquaternium 6 and Polyquaternium 7, respectively; mineral acid salts of aminoalkyl esters of homo-and co-polymers of unsaturated carboxylic acids having from 3 to 5 carbon atoms, as described in U.S. Patent 4,009,256; and cationic polyacrylamides as described in our copending UK Application No. 9403156.4 (W095/22311). Other cationic deposition aids that can be used include polysaccharide polymers, such as cationic cellulose derivatives and cationic starch derivatives. Cationic polysaccharide polymer materials suitable for use in compositions of the invention include those of the formula: A-O(R-N$^+$R$^1$R$^2$R$^3$X$^-$) wherein: A is an anhydroglucose residual group, such as starch or cellulose anhydroglucose residual, R is an alkylene oxyalklene, polyoxyalkylene, or hydroxyalkylene group, or combination thereof, R', R~' and R3 independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R', R 2 and R') preferably being about 20 or less, and X is an anionic counterion , as previously described. Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200. Other cationic deposition aids that can be used include cationic guar gum derivatives, such as guar hydroxypropyltrimonium chloride (Commercially available from Rhodia Novecare in their Jaguar trademark series). Other materials include quaternary nitrogen-containing cellulose ethers (e.g., as described in U.S. Patent 3,962,418) and copolymers of etherified cellulose and starch (e.g., as described in U.S. Patent 3,958,581).

The personal care composition may contain a foam boosting agent. A foam booster is an agent which increases the amount of foam available from a system at a constant molar concentration of surfactant, in contrast to a foam stabilizer which delays the collapse of a foam. Foam building is provided by adding to the aqueous media, a foam boosting effective amount of a foam boosting. The foam boosting agent is preferably selected from the group consisting of fatty acid alkanolamides and amine oxides. The fatty acid alkanolamides are exemplified by isostearic acid diethanolamide, lauric acid diethanolamide, capric acid diethanolamide, coconut fatty acid diethanolamide, linoleic acid diethanolamide, myristic acid diethanolamide, oleic acid diethanolamide, stearic acid diethanolamide, coconut fatty acid monoethanolamide, oleic acid monoisopropanolamide, and lauric acid monoisopropanolamide. The amine oxides are exemplified by N-cocodimethylamine oxide, N-lauryl dimethylamine oxide, N-myristyl dimethylamine oxide, N-stearyl dimethylamine oxide, N-cocamidopropyl dimethylamine oxide, N-tallowamidopropyl dimethylamine oxide, bis(2-hydroxyethyl) C12-15 alkoxypropylamine oxide. Preferably a foam booster is selected from the group consisting of lauric acid diethanolamide, N-lauryl dimethylamine oxide, coconut acid diethanolamide, myristic acid diethanolamide, and oleic acid diethanolamide. The foam boosting agent is preferably present in the shampoo compositions of this invention in an amount from about 1 to 15 wt% and more preferably about 2 to 10 wt% based on the total weight of the composition. The composition may further comprise a polyalkylene glycol to improve lather performance. Concentration of the polyalkylene glycol in the shampoo composition may range from about 0.01 % to about 5%, preferably from about 0.05% to about 3%, and more preferably from about 0. 1 % to about 2%, by weight of the composition. The optional polyalkylene glycols are characterized by the general formula: H(OCH2CHR)n-OH wherein R is selected from the group consisting of H, methyl, and mixtures thereof. When R is H, these materials are polymers of ethylene oxide, which are also known as polyethylene oxides, polyoxyethylenes, and polyethylene glycols. When R is methyl, these materials are polymers of propylene oxide, which are also known as polypropylene oxides, polyoxypropylenes, and polypropylene glycols. When R is methyl, it is also understood that various positional isomers of the resulting polymers can exist. In the above structure, n has an average value of from about 1500 to about 25,000, preferably from about 2500 to about 20,000, and more preferably from about 3500 to about 15,000. Polyethylene glycol polymers useful herein are PEG-2M wherein R equals H and n has an average value of about 2,000 (PEG-2M is also known as Polyox WSR9 N-10, which is available from Union Carbide and as PEG-2,000); PEG-5M wherein R equals H and n has an average value of about 5,000 (PEG-5M is also known as Polyox WSRO N-35 and Polyox WSRS N-80, both available from Union Carbide and as PEG-5,000 and Polyethylene Glycol 300,000); PEG-7M wherein R equals H and n has an average value of about 7,000 (PEG-7M is also known as Polyox WSRO N-750 available from Union Carbide); PEG-9M wherein R equals H and n has an average value of about 9,000 (PEG 9-M is also known as Polyox WSRS N-3333 available from Union Carbide); and PEG14 M wherein R equals H and n has an average value of about 14,000 (PEG-14M is also known as Polyox WSRO N-3000 available from Union Carbide). Other useful polymers include the polypropylene glycols and mixed polyethylene/polypropylene glycols.

The personal care composition may contain a suspending agent at concentrations effective for suspending the preferred silicone conditioning agent, or other water-insoluble material, in dispersed form in the shampoo compositions. Such concentrations range from about 0.1 % to about 10%, preferably from about 0.3% to about 5.0%, by weight of the shampoo compositions. Suspending agents include crystalline suspending agents which can be categorized as acyl derivatives, long chain amine oxides, and mixtures thereof, concentrations of which range from about 0.1% to about 5.0%, preferably from about 0.5% to about 3.0%, by weight of the shampoo compositions. These suspending agents are described in U.S. Patent 4.741,855. These preferred suspending agents include ethylene glycol esters of fatty acids preferably having from about 16 to about 22 carbon atoms. More preferred are the ethylene glycol stearates, both mono and distearate, but particularly the distearate containing less than about 7% of the mono stearate. Other suitable suspending agents include alkanol amides of fatty acids, preferably having from about 16 to about 22 carbon atoms, more preferably about 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); glyceryl esters (e.g., glyceryl distearate) and long chain esters of long chain alkanol amides (e.g., stearamide diethanolamide distearate, stearamide monoethanolamide stearate). Long chain acyl derivatives, ethylene glycol esters of long chain carboxylic acids, long chain amine oxides, and alkanol amides of long chain carboxylic acids in addition to the preferred materials listed above may be used as suspending agents. For example, it is contemplated that suspending agents with long chain hydrocarbyls having C8-C22 chains may be used. Other long chain acyl derivatives suitable for use as suspending agents include N,N-dihydrocarbyl amido benzoic acid and soluble salts thereof (e.g., Na, K), particularly N,N-di(hydrogenated) C16, C18 and tallow amido benzoic acid species of this family, which are commercially available from Stepan Company (Northfield, Illinois, USA). Examples of suitable long chain amine oxides for use as suspending agents include alkyl (C16-C22) dimethyl amine oxides, e.g., stearyl dimethyl amine oxide. Other suitable suspending agents include xanthan gum at concentrations ranging from about 0.3% to about 3%, preferably from about 0.4% to about 1.2%, by weight of the shampoo compositions. The use of xanthan gum as a suspending agent in silicone containing shampoo compositions is described, for example, in U.S. Patent 4,788,006. Combinations of long chain acyl derivatives and xanthan gum may also be used as a suspending agent in the shampoo compositions. Such combinations are described in U.S. Patent 4,704,272. Other suitable suspending agents include carboxyvinyl polymers. Preferred among these polymers are the copolymers of acrylic acid crosslinked with polyallylsucrose as described in U.S. Patent 2,798,053.

Examples of these polymers include Carbopol 934, 940, 941, and 956. available from B. F. Goodrich Company. Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow) amine. Still other suitable suspending agents include di(hydrogenated tallow)phthalic acid amide, and crosslinked maleic anhydride-methyl vinyl ether copolymer. Other suitable suspending agents may be used in the shampoo compositions, including those that can impart a gel-like viscosity to the composition, such as water soluble or colloidally water soluble polymers like cellulose ethers (e.g., methylcellulose, hydroxybutyl methylcellulose, hyroxypropylcellulose, hydroxypropyl methylcellulose, hydroxyethyl ethylcellulose and hydroxyethylcellulose), guar gum, polyvinyl alcohol, polyvinyl pyrrolidone, hydroxypropyl guar gum, starch and starch derivatives, and other thickeners, viscosity modifiers gelling agents, etc.

The personal care composition may contain one or more water-soluble emollients including, but not limited to, lower molecular weight aliphatic diols such as propylene glycol and butylene glycol; polyols such as glycerine and sorbitol; and polyoxyethylene polymers such as polyethylene glycol 200. The specific type and amount of water soluble emollient (s) employed will vary depending on the desired aesthetic characteristics of the composition, and is readily determined by one skilled in the art.

The personal care composition may contain various oils. The term "oil" as used herein refers to any material which is substantially insoluble in water. When the composition is to be used in a cosmetic or personal care product, the product components must also be cosmetically acceptable or otherwise meet the conditions of the end use product. Suitable oil components include, but are not limited to, natural oils such as coconut oil; hydrocarbons such as mineral oil and hydrogenated polyisobutene; fatty alcohols such as octyldodecanol; esters such as C12 -C15 alkyl benzoate; diesters such as propylene dipelarganate; and triesters, such as glyceryl trioctanoate and silicones especially cyclomethicone and dimethicone and mixtures thereof. The composition of the invention also contains oils, preferably a mixture of low viscosity and high viscosity oils. Suitable low viscosity oils have a viscosity of 5 to 100 mPa.s at 25°C, and are generally esters having the structure RCO-OR' wherein RCO represents the carboxylic acid radical and wherein OR' is an alcohol residue. Examples of these low viscosity oils include isotridecyl isononanoate, PEG-4 diheptanoate, isostearyl neopentanoate, tridecyl neopentanoate, cetyl octanoate, cetyl palmitate, cetyl ricinoleate, cetyl stearate, cetyl myristate, coco-dicaprylate/caprate, decyl isostearate, isodecyl oleate, isodecyl neopentanoate, isohexyl neopentanoate, octyl palmitate, dioctyl malate, tridecyl octanoate, myristyl myristate, octododecanol, or mixtures of octyldodecanol, acetylated lanolin alcohol, cetyl acetate, isododecanol, polyglyceryl-3-diisostearate, or mixtures thereof. The high viscosity surface oils generally have a viscosity of 200-1,000,000 mPa.s at 25°C, preferably a viscosity of 100,000-250,000 mPa.s. Surface oils include castor oil, lanolin and lanolin derivatives, triisocetyl citrate, sorbitan sesquioleate, C10-18 triglycerides, caprylic/capric/triglycerides, coconut oil, corn oil, cottonseed oil, glyceryl triacetyl hydroxystearate, glyceryl triacetyl ricinoleate, glyceryl trioctanoate, hydrogenated castor oil, linseed oil, mink oil, olive oil, palm oil, illipe butter, rapeseed oil, soybean oil, sunflower seed oil, tallow, tricaprin, trihydroxystearin, triisostearin, trilaurin, trilinolein, trimyristin, triolein, tripalmitin, tristearin, walnut oil, wheat germ oil, cholesterol, or mixtures thereof. The suggested ratio of low viscosity to high viscosity oils in the oil phase is 1:15 to 15:1, preferably 1:10 to 10:1 respectively. The preferred formulation of the invention comprises 1 to 20% of a mixture of low viscosity and high viscosity surface oils.

Mention may be made, among the optional other non-silicone fatty substances, of mineral oils, such as liquid paraffin or liquid petroleum, of animal oils, such as perhydrosqualene or arara oil, or alternatively of vegatable oils, such as sweet almond, calophyllum, palm, castor, avocado, jojaba, olive or cereal germ oil. It is also possible to use esters of lanolic acid, of oleic acid, of lauric acid, of stearic acid or of myristic acid, for example; alcohols, such as oleyl alcohol, linoleyl or linolenyl alcohol, isostearyl alcohol or octyldodecanol; or acetylglycerides, octanoates, decanoates or ricinoleates of alcohols or of polyalcohols. It is alternatively possible to use hydrogenated oils which are solid at 25°C, such as hydrogenated castor, palm or coconut oils, or hydrogenated tallow; mono-, di-, tri- or sucroglycerides; lanolins; or fatty esters which are solid at 25°C.

The personal care composition may contain various waxes. The waxes or wax-like materials generally have a melting point range of 35 to120°C at atmospheric pressure. Waxes in this category include synthetic wax, ceresin, paraffin, ozokerite, illipe butter, beeswax, carnauba, microcrystalline, lanolin, lanolin derivatives, candelilla, cocoa butter, shellac wax, spermaceti, bran wax, capok wax, sugar cane wax, montan wax, whale wax, bayberry wax, or mixtures thereof. The preferred formulation of the invention comprises about 10-30% of a mixture of waxes. Mention may be made, among the waxes capable of begin used as non-silicone fatty substances, of animal waxes, such as beeswax; vegetable waxes, such as carnauba, candelilla, ouricury or japan wax or cork fibre or sugarcane waxes; mineral waxes, for example paraffin or lignite wax or microcrystalline waxes or ozokerites; synthetic waxes, including polyethylene waxes, and waxes obtained by the Fischer-Tropsch synthesis. Mention may be made, among the silicone waxes, of polymethylsiloxane alkyls, alkoxys and/or esters.

The personal care composition may contain various powders. The powder component of the invention can be generally defined as dry, particulate matter having a particle size of 0.02-50 microns. The particulate matter may be colored or non-colored (for example white). Suitable powders include bismuth oxychloride, titanated mica, fumed silica, spherical silica beads, polymethylmethacrylate beads, micronized teflon, boron nitride, acrylate polymers, aluminum silicate, aluminum starch octenylsuccinate, bentonite, calcium silicate, cellulose, chalk, corn starch, distomaceous earth, fuller's earth, glyceryl starch, hectorite, hydrated silica, kaolin, magnesium aluminum silicate, magnesium carbonate, magnesium hydroxide, magnesium oxide, magnesium silicate, magnesium trisilicate, maltodextrin, montmorillonite, microcrystalline cellulose, rice starch, silica, talc, mica, titanium dioxide, zinc laurate, zinc myristate, zinc neodecanoate, zinc rosinate, zinc stearate, polyethylene, alumina, attapulgite, calcium carbonate, calcium silicate, dextran, kaolin, nylon, silica silylate, silk powder, serecite, soy flour, tin oxide, titanium hydroxide, trimagnesium phosphate, walnut shell powder, or mixtures thereof. The above mentioned powders may be surface treated with lecithin, amino acids, mineral oil, silicone oil, or various other agents either alone or in combination, which coat the powder surface and render the particles hydrophobic in nature. The powder component also comprises various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows, etc. Inorganic pigments generally consist of insoluble metallic salts of certified color additives, referred to as the Lakes or iron oxides.

A pulverulent colouring agent, such as carbon black, chromium or iron oxides, ultramarines, manganese pyrophosphate, iron blue, and titanium dioxide, pearlescent agents, generally used as a mixture with coloured pigments, or some organic dyes, generally used as a mixture with coloured pigments and commonly used in the cosmetics industry, can be added to the composition. In general, these colouring agents can be present in an amount by weight from 0 to 20% with respect to the weight of the final composition.

Pulverulent inorganic or organic fillers can also be added, generally in an amount by weight from 0 to 40% with respect to the weight of the final composition. These pulverulent fillers can be chosen from talc, micas, kaolin, zinc or titanium oxides, calcium or magnesium carbonates, silica, spherical titanium dioxide, glass or ceramic beads, metal soaps derived from carboxylic acids having 8-22 carbon atoms, non-expanded synthetic polymer powders, expanded powders and powders from natural organic compounds, such as cereal starches, which may or may not be crosslinked. The fillers may preferably be present in a proportion of from 0 to 35% of the total weight of the composition, more preferably 5 to 15%. Mention may be made in particular of talc, mica, silica, kaolin, nylon powders (in particular ORGASOL), polyethylene powders, Teflon, starch, boron nitride, copolymer microspheres such as EXPANCEL (Nobel Industrie), polytrap and silicone resin microbeads (TOSPEARL from Toshiba, for example).

The personal care composition may contain sunscreens. These include those which absorb ultraviolet light between about 290-320 nanometers (the UV-B region) such as, but not exclusively, para-aminobenzoic acid derivatives and cinnamates such as octyl methoxycinnamate and those which absorb ultraviolet light in the range of 320-400 nanometers (the UV-A region) such is benzophenones and butyl methoxy dibenzoylmethane. Some additional examples of sunscreen chemicals which may be employed in accordance with the present invention are 2-ethoxyethyl p-methoxycinnamate; menthyl anthranilate; homomenthyl salicylate; glyceryl p-aminobenzoate; isobutyl p-aminobenzoate; isoamyl p-dimethylaminobenzoate; 2-hydroxy-4-methoxybenzophenones sulfonic acid; 2,2'-dihydroxy-4-methoxybenzophenone; 2-hydroxy-4-methoxybenzophenone; 4-mono and 4-bis(3-hydroxy-propyl)amino isomers of ethyl benzoate; and 2-ethylhexyl p-dimethylaminobenzoate. As hydrophilic screening agents which can be used in the invention, mention may be made of those described in Application EP-A-678,292. These hydrophilic screening agents are those containing at least one carboxylic or better still sulphonic acid radical. This acid radical can be in free form or in partially or totally neutralized form. It is possible to use one or more hydrophilic screening agents containing acid functionality. As examples of acidic screening agents containing at least one $SO_3H$ group, mention may be made more particularly of 3-benzylidine-2-camphorsulphonic derivatives. A particularly preferred compound is benzene-1,4-(di(3-methylidenecamphor-10-sulphonic acid)]. This screening agent is a broad-band screening agent capable of absorbing ultraviolet rays with wavelengths of between 280 nm and 400 nm, with absorption maxima of between 320 nm and 400 nm, in particular at about 345 nm. It is used in acid form or salified with a base chosen from triethanolamine, sodium hydroxide and potassium hydroxide. In addition, it can be in cis or trans form. This screening agent is known under the trade name Mexoryl SX. Other specific examples are 4-(3-methylidenecamphor)-benzenesulphonic acid, 3-benzylidenecamphor-10-sulphonic acid, 2-methyl-5-(3-methylidenecamphor)benzenesulphonic acid, 2-chloro-5-(3-methylidenecamphor)-benzenesulphonic acid, 3-(4-methyl)benzylidenecamphor-10-sulphonic acid, (3-t-butyl-2-hydroxy-5-methyl)benzylidenecamphor-10-sulphonic acid, (3-t-butyl-2-hydroxy-5-methoxy)benzylidenecamphor-10-sulphonic acid, (3,5-di-tert-butyl-4-hydroxy)benzylidenecamphor-10-sulphonic acid, 2-methoxy-5-(3-methylidenecamphor)benzenesulphonic acid, 3-(4,5-methylenedioxy)benzylidenecamphor-1 0-sulphonic acid, 3-(4-methoxy)benzylidenecamphor-10-sulphonic acid, 3-(4,5-dimethoxy)benzylidenecamphor-10-sulphonic acid, 3-(4-n-butoxy)benzylidenecamphor-10-sulphonic acid, 3-(4-n-butoxy-5-methoxy)benzylidenecamphor-10-sulphonic acid, 2-[4-(camphormethylidene)phenyl]benzimidazole-5-sulphonic acid. Suitable compounds are described in U.S. Pat. No. 4,585,597, patent applications FR 2,236,515, 2,282,426, 2,645,148, 2,430,938 and 2,592,380. The screening agent containing a sulphonic group can also be a sulphonic derivative of benzophenone or 2-phenylbenzimidazole-5-sulphonic acid, having excellent photoprotective power in the UV-B radiation range and is sold under the trade name "Eusolex 232" by the company Merck, benzene-1 ,4-di(benzimidazol-2-yl-5-sulphonic acid), benzene-1,4-di(benzoxazol-2-yl-5-sulphonic acid). The hydrophilic screening agent(s) can be present in the final composition according to the invention in a content which can range from 0.1 to 20%, preferably from 0.2 to 10%, by weight relative to the total weight of the composition. As lipophilic screening agents which can be used in the invention, mention may be made advantageously of the family of screening agents derived from dibenzoylmethane and more especially 4-tert-butyl-4'-methoxydibenzoylmethane, which effectively have a high intrinsic power of absorption. These dibenzoylmethane derivatives, which are products that are well known per se as UV-A active screening agents, are described in particular in French patent applications FR-A-2,326,405 and FR-A-2,440,933, as well as in European patent application EP-A-0,114,607; 4-(tert-butyl)-4'-methoxydibenzoylmethane is moreover currently sold under the trade name "Parsol 1789" by the company Givaudan. Another dibenzoylmethane derivative which is preferred according to the present invention is 4-sopropyldibenzoylmethane, this screening agent being sold under the name "Eusolex 8020" by the company Merck. Similarly octocrylene, a liquid lipophilic screening agent that is already known for its activity in the UV-B range is commercially available, and is sold in particular under the name "Uvinul N 539" by the company BASF. As another lipophilic (or liposoluble) screening agent which can be used in the invention, mention may also be made of p-ethylbenzylidenecamphor, which is also known as a UV-B absorber and is sold in particular under the trade name "Eusolex 6300" by the company Merck. The lipophilic screening agent(s) can be present in the composition according to the invention in a content which can range from 0.5 to 30%, preferably from 0.5 to 20%, of the total weight of the composition. Other examples of lipophilic or hydrophilic organic screening agents are given in particular in patent application EP-A-0,487,404. The cosmetic and/or dermatological compositions according to the invention can also contain pigments or alternatively nanopigments (average primary particle size: generally between 5 nm and 100 nm, preferably between 10 and 50 nm) of coated or uncoated metal oxides, such as, for example, nanopigments of titanium oxide (amorphous or crystallized in rutile and/or anatase form), of iron oxide, of zinc oxide, of zirconium oxide or of cerium oxide, which are all photoprotective agents that are well known per se and which act by physically blocking (reflection and/or scattering) UV radiation. Standard coating agents are, moreover, alumina and/or aluminium stearate, and silicones. Such coated or uncoated metal oxide nanopigments are described in particular in patent applications EP-A-0,518,772 and EP-A-0,518,773.

Thickening agent may be added to provide a convenient viscosity. For example, viscosities within the range of 500 to 25,000 $mm^2$/s at 25°C or more alternatively in the range of 3,000 to 7,000 $mm^2$/s are usually suitable. Suitable thickening agents are exemplified by sodium alginate, gum arabic, polyoxyethylene, guar gum, hydroxypropyl guar gum, ethoxylated alcohols, such as laureth-4 or polyethylene glycol 400, cellulose derivatives exemplified by methylcellulose, methylhydroxypropylcellulose, hydroxypropylcellulose, polypropylhydroxyethylcellulose, starch, and starch derivatives exemplified by hydroxyethylamylose and starch amylose, locust bean gum, electrolytes exemplified by sodium chloride and ammonium chloride, and saccharides such as fructose and glucose, and derivatives of saccharides such as PEG-120 methyl glucose diolate or mixtures of 2 or more of these. Alternatively the thickening agent is selected from cellulose derivatives, saccharide derivatives, and electrolytes, or from a combination of two or more of the above thickening agents exemplified by a combination of a cellulose derivative and any electrolyte, and a starch derivative and any electrolyte. The thickening agent, where used is present in the shampoo compositions of this invention in an amount sufficient to provide a viscosity in the final shampoo composition of from 500 to 25,000 $mm^2/s$. Alternatively the thickening agent is present in an amount from about 0.05 to 10 wt% and alternatively 0.05 to 5 wt% based on the total weight of the composition.

Stabilizing agents can be used in the water phase of the compositions. Suitable water phase stabilizing agents can include alone or in combination one or more electrolytes, polyols, alcohols such as ethyl alcohol, and hydrocolloids. Typical electrolytes are alkali metal salts and alkaline earth salts, especially the chloride, borate, citrate, and sulfate salts of sodium, potassium, calcium and magnesium, as well as aluminum chlorohydrate, and polyelectrolytes, especially hyaluronic acid and sodium hyaluronate. When the stabilizing agent is, or includes, an electrolyte, it amounts to about 0.1 to 5 wt % and more alternatively 0.5 to 3 wt % of the total composition. The hydrocolloids include gums, such as Xantham gum or Veegum and thickening agents, such as carboxymethyl cellulose. Polyols, such as glycerine, glycols, and sorbitols can also be used. Alternative polyols are glycerine, propylene glycol, sorbitol and butylene glycol. If a large amount of a polyol is used, one need not add the electrolyte. However, it is typical to use a combination of an electrolyte, a polyol and an hydrocolloid to stabilize the water phase, e.g. magnesium sulfate, butylene glycol and Xantham gum.

Examples

All measurements and experiments were conducted at 23°C, unless indicated otherwise.

Mean particle size was determined using a *Malvern Mastersizer 2000.* Rheological properties were determined using a *TA Instruments ARES* rheometer equipped with cone-and-plate.

Example 1

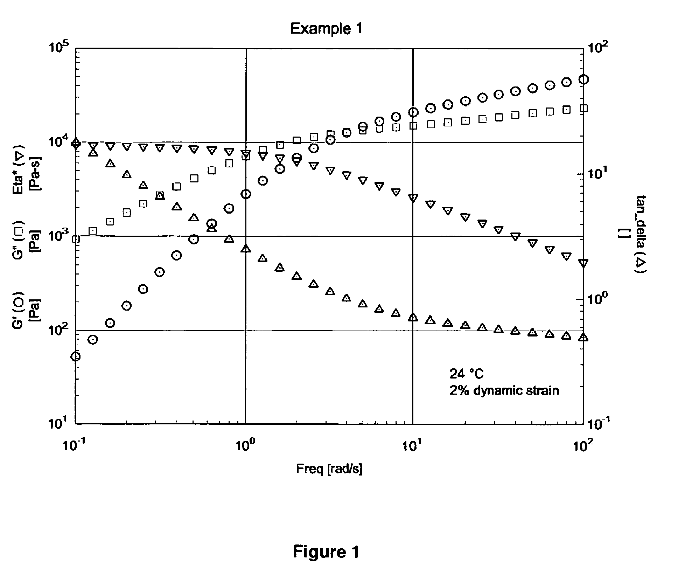

First, 50 g of a $Me_2SiOH$ terminated dimethylpolysiloxane having a viscosity of approximately 50,000 mPa sec. (cP) and a number average molecular weight of approximately 61,000 was weighed into a max 100 cup followed by 0.10g of a 50% by weight dispersion of boric acid in trimethylsiloxy-capped polydimethylsiloxane fluid having an approximate kinematic viscosity of 1000 cSt. The cup was closed and placed into a Speedmixer® DAC 150 mixer and the cup was spun for 20 seconds at maximum speed. The cup was allowed to remain undisturbed for 15 minutes after which it was spun again for 20 seconds at maximum speed. After an additional 10 minutes at rest, the cup was spun again for 20 seconds. Immediately following the previous spinning, 0.75 g of lauryl alcohol (4) ethoxylate (Brij@ 30) was added followed by 1.5g of a 72 % aqueous solution of lauryl alcohol (23) ethoxylate (Brij@ 35L) and 0.60 g of deionized (DI) water. The cup was closed and spun in the Speedmixer® for 20 seconds. The contents of the cup had formed a mass having a thick gel-like consistence and the walls of the cup were scraped with a spatula. The cup and its contents were spun again for 20 seconds. The thick phase composition was diluted incrementally with a total of 31 g of DI water. The first increment was 5g followed by another 5g then 10 g followed with a final 11 g. The cup was spun for 18 seconds at full speed in the SpeedMixer® after each incremental addition of water. The resulting final composition was a milky-white liquid of low viscosity and consisted of an oil/water emulsion of approximately 60% by weight polydimethylsiloxane containing boron oxide and having a mean particle size of approximately 0.75 $\mu$m. The following day, a 20g portion of the emulsion was poured into a Petri dish and allowed to evaporate and at ambient conditions for 24 hours. The resulting polymer from the emulsion was inspected and found to have increased in viscosity substantially from that of the starting silicone polymer. Viscosity of the polymer from the emulsion was determined using a rheometer and found to have a zero-shear-rate viscosity of approximately $10^7$ cP (@$10^{-1}$ sec.$^{-1}$ & 24°C), as summarized in figure 1. These results show the viscosity of the siloxane polymer from the emulsion was significantly higher than the viscosity of the starting siloxane polymer (approximately 5 x $10^4$ cP).

Example 2

Using the same procedure as described in Example 1, a composition prepared from 50g of a $Me_2SiOH$ terminated dimethylpolysiloxane having a viscosity of approximately 50,000 mPa sec. (cP) and a number average molecular weight of approximately 61,000, 0.11 g of a 50% by weight dispersion of boric acid in trimethylsiloxy-capped polydimethylsiloxane fluid having an approximate kinematic viscosity of 1000 cSt was emulsified using 0.75g of lauryl alcohol (4) ethoxylate (Brij® 30, 1.5g of a 72 % aqueous solution of lauryl alcohol (23) ethoxylate (Brij@ 35L) and 0.60g of deionized (DI) water. The emulsion thick phase was diluted incrementally with a total of 31 g of DI water using the same procedure of example 1. Mean particle size of the emulsion was approximately 0.75 $\mu$m. Viscosity of the polymer from the emulsion was determined using a rheometer and found to have a viscosity of approximately 6 x $10^7$ cP (@$10^{-1}$ sec.$^{-1}$ & 24°C).

Example 3

Using the same procedure and quantities as described in Example 2 except for the amount of boron oxide dispersion in polydimethylsiloxane used, an emulsion was prepared. This emulsion was prepared using 50g of a $Me_2SiOH$ terminated dimethylpolysiloxane and 0.40g of boron oxide/polydimethylsiloxane dispersion. Mean particle size of the emulsion was approximately 0.75 $\mu$m.

Example 4

Using the procedure as described in Example 1, a composition prepared from 50g SiOH functional polydimethylsiloxane having a viscosity of approximately 50,000 mPa·sec. (cP) and a number average molecular weight of approximately 61,000, 0.50g of a 50% by weight dispersion of boric acid in trimethylsiloxy-capped polydimethylsiloxane fluid having an approximate kinematic viscosity of 1000 cSt was emulsified using 1.25g of 60% by weight aqueous sodium secondary alkyl sodium sulfonate (Hostapur® SAS-60) and 5.0g of water. The resulting thick phase composition was diluted with 27g of DI water incrementally as described in Example 1. The resulting emulsion consisted of an approximately 60% solids anionic emulsion of PDMS containing 0.5% $B_2O_3$. Removal of water from this emulsion resulted in a high viscosity polymer.

Example 5

Using the procedure as described in Example 1, a composition prepared from 50g SiOH functional polydimethylsiloxane having a viscosity of approximately 50,000 mPa·sec. (cP) and a number average molecular weight of approximately 61,000, 0.50g of a 50% by weight dispersion of boric oxide in trimethylsiloxy-capped polydimethylsiloxane fluid having an approximate kinematic viscosity of 1000 cSt was emulsified using 3.3g of a 30% aqueous solution of cetyltrimethylammonium chloride (CETAC® 30) and 0g of water. The resulting thick phase was diluted with 30g of DI water incrementally as described in Example 1. The resulting emulsion consisted of an approximately 60% cationic emulsion of PDMS containing 500ppm $B_2O_3$. Removal of water from this emulsion resulted in a film of polymer that had a rubbery consistency.

Example 6

The emulsions from Example 1 and 2 were evaluated on hair. These emulsions were incorporated into shampoos, applied to hair tresses and wet and dry combing forces using an Instron tensile tester were obtained for the tresses. The preparation of tresses and combing procedure are detailed below. The conditioning performance of these two emulsions was comparable to that of DC 1785 Emulsion ($\sim 10^6$ cP) and HMW 2220 Emulsion ($\sim 12 \times 10^7$ cP), which were used as controls in these experiments. The shampoo compositions are shown in Table I and wet and dry combing results can be found in Table II.

**TABLE I**

|  |  |  |  | Si Control | Si Control |
|---|---|---|---|---|---|
| Conditioning Shampoo Formulation | | Target % Active | 2 | 2 | 2 |
|  |  |  | Example 1 or 2 | DC 1785 | HMW 2220 |
|  |  | Polymer % Active | 60 | 60 | 60 |

(continued)

| | | Ingredients | | wt % | wt % | wt % |
|---|---|---|---|---|---|---|
| Phase A | | | Polyquatemium-10 | 0.30% | 0.30% | 0.30% |
| | | | Tetrasodium EDTA | 0.20% | 0.20% | 0.20% |
| | Standapol ES-3 | | Sodium Lauryl Ether Sulfate (30% active) | 30 0% | 30 0% | 30.0% |
| | Crothix | | PEG-150 Pentaerythrityl Tetrastearate | 0.50% | 0.50% | 0.50% |
| | Water | | | 55.3% | 55.3% | 55.3% |
| Phase B | Monamid 705 | | Cocamide DEA | 3 00% | 3.00% | 3.00% |
| | Velvetex BA-35 | | Cocamidopropyl Betaine (30% active) | 7.00% | 7.00% | 7.00% |
| Phase C* | Active | | Si Test Material | 3.3% | 3.3% | 3.3% |
| Phase D | Water | | | q s. | q.s. | q s. |
| | Glydant | | DMDM Hydantoin | 0 40% | 0.40% | 0 40% |
| % | | | | 44.7% | 44.7% | 44.7% |
| | | | TOTAL | 100 | 100 | 100 |

**Procedure**

Add EDTA, then PQ-10 to water and SLES while mixing

Heat/Mix to 70 deg C

Add Crothix and continue to mix until fully melted

Turn off heat and reduce speed to 400 rpm

Add Cocamide DEA and Cocamidopropyl Betaine in order

Cool to 40 deg C

Add Silicone Emulsion

Add Glydant and mix until uniform

Compensate for water loss due to heating

**TABLE II**

| Wet and Dry Combing Results | | | | |
|---|---|---|---|---|
| Sample | Reduction in Dry Combing Force (%) | Reduction in Wet Combing Force (%) | Average Combing Load-Dry | Average Combing Load- Wet |
| Example 1 | 49.7 | 66.7 | 0.028 | 0.201 |
| Example 2 | 26.5 | 59.0 | 0.040 | 0.269 |
| DC 1785 | 47.1 | 58.5 | 0.031 | 0.318 |
| HMW 2220 | 51.8 | 50.5 | 0.029 | 0.286 |

**Preparation of Hair Samples**

Slightly bleached European human hair from DeMeo Brothers, Inc. was used for testing performance of the hair shampoos. A master hand of hair about eight inches in length was subdivided into a series of individual hair tresses. Each tress weighed about 2.5 grams. A 0.5 inch section of the root end of the hair was trimmed off and the remaining hair was glued to a 2 inch by 2 inch plastic tab using DUCO CEMENT ®. The cement was allowed to dry, and the hair tress was combed and trimmed to a length so that six inches of hair extended below the bottom of the plastic tab. A hole was punched in middle of tab about one fourth inch from the top. Each tress was rinsed for 15 seconds under 40°C tap water. Using a pipette, 1.0 ml of a 9% (active) sodium lauryl sulfate solution was applied and lathered through the tress for 30 seconds. The tress was rinsed for 30 seconds under running water. Excess water was removed from the tress by passing the tress between the index and middle fingers. The tresses were placed on a tray covered with paper towels and dried overnight. Each tress was hand combed three times with the narrow teeth of an ACE ® comb and had its baseline comb loading established using the INSTRON "WET" and the INSTRON "DRY" COMBING procedures.

For tests involving shampoos, the hair tress was rinsed with tap water for 30 seconds at 40°C. The test conditioner was applied to the tress in the amount of 0.8 g, and the tress was stroked for 30 seconds. The tress was rinsed for 30 seconds under tap water at 40°C. The excess water was removed by pulling the tress through the index and middle fingers. The tresses were allowed to dry separately on a paper towel overnight at room temperature. The tresses were combed once before performing the INSTRON study.

**Combing Test Procedure**

Ease of wet or dry combing was determined using an INSTRON strain gauge to measure the force required to comb the hair. Conditioning performance is based on the ability of a particular hair treatment formulation such as a shampoo or a hair conditioner to reduce the force required to comb the hair with the INSTRON strain gauge. The force is reported as Average Combing Load (ACL). The lower ACL value, the better the conditioning effect imparted by the formulation being tested. Typically, ACL baselines are initially established with "untreated" tresses that have only been washed with 9% (active) sodium lauryl sulfate solution. The effectiveness of a treatment can be expressed as the ACL of the treated tress or the percent reduction in ACL which is calculated using the formula:

$$((untreated\ hair\ ACL\text{-}treated\ hair\ ACL)/untreated\ hair\ ACL)*100$$

**INSTRON Wet Combing Method**

The hair was first wet by dipping it in distilled water and then the hair was detangled by combing the tress three times. The tress was then retangled by dipping in distilled water three times. The excess water was removed by passing the tress through index and middle fingers twice. The tress was then placed on a hanger and INSTRON combed. The "retangle" and "INSTRON combing" steps were repeated until five data points were collected for each tress. An average combing force of three tresses was measured for each treatment.

**INSTRON Dry Combing Method**

The hair was detangled by combing the tress 3 times. Then the hair was retangled by swirling the tress clockwise 3 times and counter clockwise 3 times. The tress was then placed on the hanger and INSTRON combed. The "retangle" and "Instron combing" steps were repeated until five data points were collected for each tress. An average combing force of three tresses was measured for each treatment.

**Claims**

1. An emulsion of a boron crosslinked organopolysiloxane having a viscosity of at least 1000 mPa·s at 24 °C wherein the emulsion is obtainable by:

    I) forming a mixture of;

        A) a silanol functional organopolysiloxane,
        B) a boron compound,
        C) an emulsifier,

    II) admixing water to the mixture from step I) to form an emulsion,
    III) optionally, further shear mixing the emulsion.

2. The emulsion according to claim 1 wherein the mixture of step I contains 50 - 99 weight percent A),

    0.05 - 30 weight percent B),

0.10 - 40 weight percent C),
with the proviso the sum of A), B), and C) is 100 weight percent.

3.  The emulsion according to claim 1 wherein the emulsion is a water continuous emulsion having a dispersed phase comprising a boron crosslinked organopolysiloxane.

4.  The emulsion according to claim 1 wherein the organopolysiloxane is a silanol terminated polydimethylsiloxane.

5.  The emulsion according to claim 1 wherein the boron compound is dispersed in a trimethylsiloxy terminated polydimethylsiloxane.

6.  The emulsion according to claim 1 wherein the boron compound is $B_2O_3$ or $H_3BO_3$.

7.  The emulsion according to claim 1 wherein the emulsifier is a mixture of nonionic surfactants.

8.  The emulsion according to claim 7 wherein the nonionic surfactants are polyoxyethylene fatty alcohols.

9.  The emulsion according to claim 7 wherein the nonionic surfactants have a combined HLB value of 8 - 15.

10. The emulsion according to claim 7 wherein the nonionic surfactant mixture comprises polyoxyethylene (23) lauryl ether and polyoxyethylene (4) lauryl ether.

11. The emulsion according to claim 7 wherein the nonionic surfactant mixture comprises an ethoxylated C10-Guerbet alcohol or an ethoxylated iso-C13 alcohol.

12. The emulsion according to claim 1 wherein 5 to 700 parts water are admixed for every 100 parts of the step I mixture to form the emulsion.

13. The emulsion according to claim 12 wherein the water is added in incremental portion such that each portion is less than 30 weight % of the mixture from step I.

14. A personal care composition comprising the emulsion of any one of the preceding claims.

15. The personal care composition of claim 14 wherein the personal care composition is selected from a color cosmetic, a lipstick, a foundation, a shampoo, a hair conditioner, a hair fixative, a hair styling aid, a hair colorant, a hair relaxer, a shower gel, a skin moisturizer, a skin conditioner, a body conditioner, a sun protection product, an antiperspirant, and a deodorant.

**Patentansprüche**

1.  Emulsion eines Bor-vernetzten Organopolysiloxans mit einer Viskosität von mindestens 1000 mPa·s bei 24°C, worin die Emulsion durch Folgendes erhalten werden kann:

    I) Bilden einer Mischung aus:

    A) einem Silanol-funktionellen Organopolysiloxan,
    B) einer Borverbindung,
    C) einem Emulgator,

    II) Einmischen von Wasser in die Mischung aus Schritt I), um eine Emulsion zu bilden,
    III) optional weiteres Schermischen der Emulsion.

2.  Emulsion nach Anspruch 1, worin die Mischung von Schritt I Folgendes enthält:

    50-99 Gewichtsprozent A),
    0,05-30 Gewichtsprozent B),
    0,10-40 Gewichtsprozent C),

mit der Maßgabe, dass die Summe aus A), B) und C) 100 Gewichtsprozent beträgt.

3. Emulsion nach Anspruch 1, worin die Emulsion eine wasserkontinuierliche Emulsion ist, die eine dispergierte Phase aufweist, die ein Bor-vernetztes Organopolysiloxan umfasst.

4. Emulsion nach Anspruch 1, worin das Organopolysiloxan ein Silanol-terminiertes Polydimethylsiloxan ist.

5. Emulsion nach Anspruch 1, worin die Borverbindung in einem Trimethylsiloxy-terminierten Polydimethylsiloxan dispergiert ist.

6. Emulsion nach Anspruch 1, worin die Borverbindung $B_2O_3$ oder $H_3BO_3$ ist.

7. Emulsion nach Anspruch 1, worin der Emulgator eine Mischung aus nichtionischen oberflächenaktiven Stoffen ist.

8. Emulsion nach Anspruch 7, worin die nichtionischen oberflächenaktiven Stoffe Polyoxyethylen-Fettalkohole sind.

9. Emulsion nach Anspruch 7, worin die nichtionischen oberflächenaktiven Stoffe einen gemeinsamen HLB-Wert von 8-15 aufweisen.

10. Emulsion nach Anspruch 7, worin die Mischung aus nichtionischen oberflächenaktiven Stoffen Polyoxyethylen(23)laurylether und Polyoxyethylen(4)laurylether umfasst.

11. Emulsion nach Anspruch 7, worin die Mischung aus nichtionischen oberflächenaktiven Stoffen einen ethoxylierten C10-Guerbet-Alkohol oder einen ethoxylierten Iso-C13-Alkohol umfasst.

12. Emulsion nach Anspruch 1, worin 5 bis 700 Teile Wasser für jeweils 100 Teile der Mischung von Schritt I eingemischt werden, um die Emulsion zu bilden.

13. Emulsion nach Anspruch 12, worin das Wasser schrittweise in Teilen zugegeben wird, sodass jeder Teil weniger als 30 Gewichts-% der Mischung aus Schritt 1 beträgt.

14. Körperpflegezusammensetzung, die eine Emulsion nach einem der vorstehenden Ansprüche umfasst.

15. Körperpflegezusammensetzung nach Anspruch 14, worin die Körperpflegezusammensetzung aus Folgenden ausgewählt ist: einem dekorativen Kosmetikum, einem Lippenstift, einer Grundierung, einem Shampoo, einer Haarspülung, einem Haarfixiermittel, einem Haar-Styling-Hilfsmittel, einem Haarfärbemittel, einem Haarglätter, einem Duschgel, einem Feuchtigkeitsmittel für die Haut, einem Haut-Conditioner, einem Körper-Conditioner, einem Sonnenschutzprodukt, einem Antitranspirant und einem Deodorant.

## Revendications

1. Emulsion d'un organopolysiloxane réticulé par du bore possédant une viscosité d'au moins 1000 mPa.s à 24° C, dans laquelle l'émulsion peut être obtenue par les étapes consistant à :

   I) former un mélange :

      A) d'un organopolysiloxane fonctionnel de silanol,
      B) d'un composé de bore,
      C) d'un émulsifiant,

   II) ajouter en mélangeant de l'eau au mélange de l'étape I) pour former une émulsion,
   III) éventuellement, mélanger l'émulsion davantage par cisaillement.

2. Emulsion selon la revendication 1, dans laquelle le mélange de l'étape I contient

   50 - 99 pourcent en poids de A),
   0,05 - 30 pourcent en poids de B),

0,10 - 40 pourcent en poids de C),

à la condition que la somme de A), B) et C) soit de 100 pourcent.

3. Emulsion selon la revendication 1, dans laquelle l'émulsion est une émulsion continue d'eau possédant une phase dispersée comprenant un organopolysiloxane réticulé par du bore.

4. Emulsion selon la revendication 1, dans laquelle l'organopolysiloxane est un polydiméthylsiloxane terminé par du silanol.

5. Emulsion selon la revendication 1, dans lequel le composé de bore est dispersé dans du polydiméthylsiloxane terminé par du triméthylsiloxy.

6. Emulsion selon la revendication 1, dans laquelle le composé de bore est $B_2O_3$ ou $H_3BO_3$.

7. Emulsion selon la revendication 1, dans laquelle l'émulsifiant est un mélange de tensioactifs non ioniques.

8. Emulsion selon la revendication 7, dans laquelle les tensioactifs non ioniques sont des alcools gras de polyoxyéthylène.

9. Emulsion selon la revendication 7, dans laquelle les tensioactifs non ioniques ont une valeur HLB combinée de 8 - 15.

10. Emulsion selon la revendication 7, dans laquelle le mélange de tensioactifs non ioniques comprend du lauryléther de polyoxyéthylène (23) et du lauryléther de polyoxyéthylène (4).

11. Emulsion selon la revendication 7, dans laquelle le mélange de tensioactifs non ioniques comprend un alcool de Guerbet en C10 ou un alcool iso-C13 éthoxylé.

12. Emulsion selon la revendication 1, dans laquelle de 5 à 700 parties d'eau sont ajoutées par mélange pour chaque 100 parties du mélange de l'étape I pour former une émulsion.

13. Emulsion selon la revendication 12, dans laquelle l'eau est ajoutée par portion progressive de sorte que chaque portion est inférieure à 30 % en poids du mélange de l'étape I.

14. Composition pour soins d'hygiène personnelle comprenant l'émulsion selon l'une quelconque des revendications précédentes.

15. Composition pour soins d'hygiène personnelle selon la revendication 14, dans laquelle la composition pour soins d'hygiène personnelle est sélectionnée parmi un produit cosmétique de couleur, un bâton de rouge à lèvres, un fond de teint, un shampoing, un après-shampoing, un fixateur pour cheveux, un produit de coiffage, un colorant pour cheveux, un produit défrisant pour cheveux, un gel douche, une crème hydratante pour la peau, une crème revitalisante pour la peau, une crème revitalisante pour le corps, un produit de protection solaire, un produit anti-sudorifique, et un déodorant.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61044594 A
- US 61139708 A
- US 4863985 A
- EP 0446157 A
- US 6051216 A
- US 5919441 A
- US 5981680 A
- WO 2004060271 A
- WO 2004060101 A
- WO 2004060276 A
- WO 03105801 A
- US 20030235553 A
- US 20030072730 A
- US 20030170188 A
- EP 1266647 A
- EP 1266648 A
- EP 1266653 A
- WO 03105789 A
- WO 2004000247 A
- WO 03106614 A
- WO 2004054523 A
- US 20040180032 A
- WO 2004054524 A
- US 4009256 A
- GB 9403156 A
- WO 9522311 A
- US 3962418 A
- US 3958581 A
- US 4741855 A
- US 4788006 A
- US 4704272 A
- US 2798053 A
- EP 678292 A
- US 4585597 A
- FR 2236515
- FR 2282426
- FR 2645148
- FR 2430938
- FR 2592380
- FR 2326405 A
- FR 2440933 A
- EP 0114607 A
- EP 0487404 A
- EP 0518772 A
- EP 0518773 A

**Non-patent literature cited in the description**

- CTFA Cosmetic Ingredient Directory